(19) Europäisches Patentamt
European Patent Office
Office européen des brevets

(11) Veröffentlichungsnummer: **0 233 841 A1**

# (12) EUROPÄISCHE PATENTANMELDUNG

(21) Anmeldenummer: **87810083.3**

(22) Anmeldetag: **12.02.87**

(51) Int. Cl.⁴: **C 07 D 471/18**
A 61 K 31/495
//(C07D471/18,241:00,221:00, 221:00)

(30) Priorität: **18.02.86 GB 8603957**
**18.02.86 GB 8603958**

(43) Veröffentlichungstag der Anmeldung:
**26.08.87 Patentblatt 87/35**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Arai, Tadashi, Prof. Dr.**
**50-6, 6-chome, Nogata**
**Nakano-ku Tokyo (JP)**

(54) **Chinon-Derivate und Verfahren zu ihrer Herstellung.**

(57) Es werden neue Saframycin-Derivate der Formel

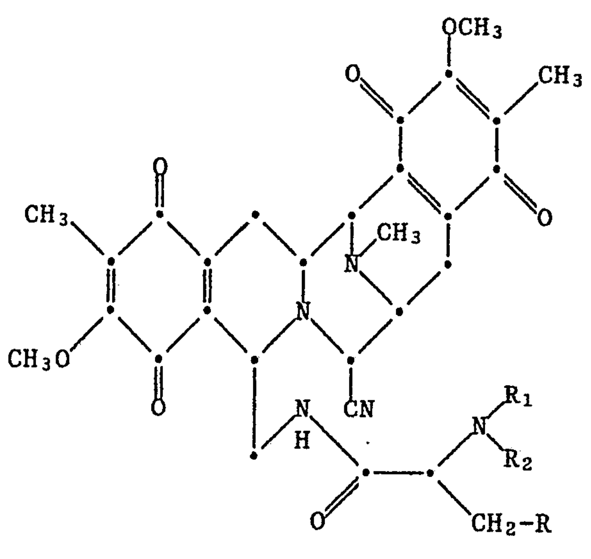

worin R Wasserstoff oder $C_1$-$C_4$-Alkyl, $R_1$ $C_1$-$C_7$-Alkyl, Aryl-$C_1$-$C_4$-alkyl oder die Acylgruppe einer organischen Säure und $R_2$ Wasserstoff oder $C_1$-$C_7$-Alkyl darstellen, und Verfahren zur Herstellung dieser Derivate beschrieben. Die neuen Saframycin-Derivate zeigen antibiotische und anti-Tumor-Wirkung.

EP 0 233 841 A1

**Beschreibung**

Chinonderivate und Verfahren zu ihrer Herstellung

Die vorliegende Erfindung betrifft neue N-substituierte Saframycin-Derivate, Verfahren zu ihrer Herstellung, pharmazeutische Zusammensetzungen, die diese neuen Verbindungen enthalten, und die Verwendung dieser neuen Derivate.

Die Europäische Patentanmeldung 173 649 und die Publikation "Directed Syntheses of New Saframycin Derivatives with Resting Cells of Streptomyces lavendulae" von Tadashi Arai et al., Antimicrobial Agents and Chemotherapy, 28, 5-11 (1985), beschreiben Saframycin A-Derivate der Formel

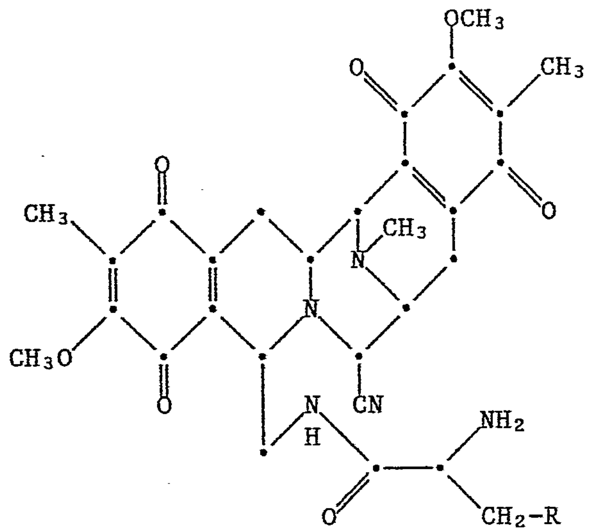

(I),

worin R Wasserstoff oder $C_1$-$C_4$-Alkyl darstellt. Das Saframycin A-Derivat, worin R Wasserstoff ist, wird Saframycin $Y_3$ genannt. Das Derivat, worin R Methyl bedeutet, heisst Saframycin $Y_{d-1}$. In diesen beiden Saframycinen hat die α-Aminogruppe in der Seitenkette die L-Konfiguration.

Gegenstand der vorliegenden Erfindung sind neue Saframycin-Derivate mit guten pharmakologischen Eigenschaften, insbesondere guten in vivo anti-Tumor-Eigenschaften.

Die vorliegende Erfindung betrifft Saframycin-Derivate der Formel

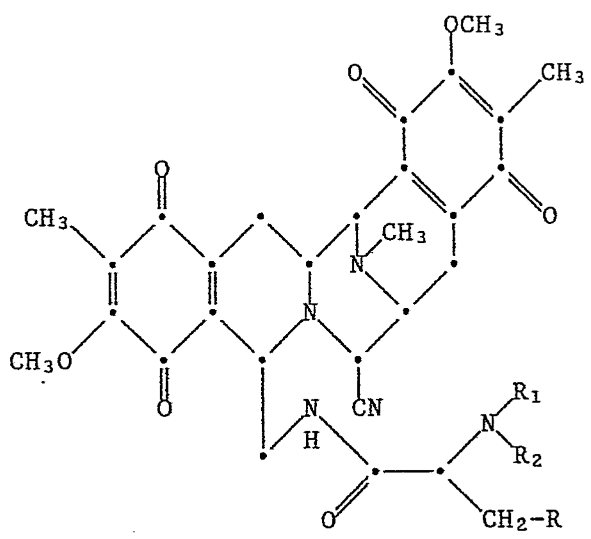

(II),

worin R Wasserstoff oder $C_1$-$C_4$-Alkyl, $R_1$ $C_1$-$C_7$-Alkyl, Aryl-$C_1$-$C_4$-alkyl oder die Acylgruppe einer organischen Säure und $R_2$ Wasserstoff oder $C_1$-$C_7$-Alkyl darstellen, und Salze dieser Verbindungen.

Im Zusammenhang mit der Beschreibung der vorliegenden Erfindung haben die allgemeinen Bezeichnungen, die vorstehend und nachstehend verwendet werden, vorzugsweise die folgenden Bedeutungen:

$C_1$-$C_4$-Alkyl ist vorzugsweise Methyl, aber auch Aethyl, Isopropyl, n-Propyl, Isobutyl, tert-Butyl oder n-Butyl.

$C_1$-$C_7$-Alkyl ist vorzugsweise $C_1$-$C_4$-Alkyl, z.B. Methyl, oder n-Pentyl, n-Hexyl oder n-Heptyl.

Aryl-$C_1$-$C_4$-alkyl ist vorzugsweise Benzyl oder Phenäthyl, worin der Phenylring durch Nitro, Amino, Halogen, beispielsweise Chlor oder Brom, Hydroxy und/oder $C_1$-$C_4$-Alkoxy, beispielsweise Methoxy, substituiert sein kann, und ist z.B. p-Nitrobenzyl, 4-Methoxybenzyl, 2-, 3- oder 4-Hydroxybenzyl oder Vanillyl.

Die Acylgruppe einer organischen Säure hat vorzugsweise bis zu 20 Kohlenstoffatome und ist in erster Linie die Acylgruppe einer Carbonsäure, eines Kohlensäurehalbesters, einer N-substituierten oder N,N-disubstituierten Carbaminsäure oder Thiocarbaminsäure, oder einer Sulfonsäure.

Solche Acylgruppen sind beispielsweise $C_1$-$C_{20}$-Alkanoyl, durch Hydroxy, Oxo oder Halogen substituiertes $C_2$-$C_7$-Alkanoyl, $C_3$-$C_{20}$-Alkenoyl, Cyclo-$C_3$-$C_7$-alkyl-$C_1$-$C_4$-alkanoyl, Cyclo-$C_3$-$C_7$-alkenyl-$C_1$-$C_4$-alkanoyl, Aroyl, $C_1$-$C_7$-Alkoxy-carbonyl, $C_1$-$C_4$-Alkyl-carbamoyl, Arylcarbamoyl, Di-$C_1$-$C_4$-alkyl-carbamoyl, $C_1$-$C_4$-Alkyl-thiocarbamoyl, Arylthiocarbamoyl, Di-$C_1$-$C_4$-alkyl-thiocarbamoyl, $C_1$-$C_7$-Alkansulfonyl oder Arylsulfonyl.

$C_1$-$C_{20}$-Alkanoyl ist vorzugsweise $C_1$-$C_7$-Alkanoyl, beispielsweise Formyl, Acetyl, Propionyl, n-Butyryl, 2-Methylpropionyl, n-Pentanoyl, 2,2-Dimethylpropionyl, 2-Methylbutyryl, 3-Methylbutyryl oder n-Hexanoyl, oder geradkettiges $C_8$-$C_{20}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl, n-Octadecanoyl oder n-Icosanoyl.

Durch Hydroxy, Oxo oder Halo substituiertes $C_2$-$C_7$-Alkanoyl ist beispielsweise Trifluoracetyl, Mono-, Di- oder Trichloracetyl, Glykoloyl, Glyceroyl, Lactoyl, Glyoxyloyl oder Pyruvoyl.

$C_3$-$C_{20}$-Alkenoyl ist vorzugsweise $C_3$-$C_7$-Alkenoyl oder geradkettiges $C_8$-$C_{20}$-Alkenoyl mit einer geraden Anzahl Kohlenstoffatome und einer, zwei oder drei Doppelbindungen, beispielsweise 2-Methylpropenoyl, cis- oder trans-2-Butenoyl, 3-Butenoyl, cis- oder trans-2-Pentenoyl, cis- oder trans-2-Hexenoyl, 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl, 9-cis-Icosenoyl, 9-cis-12-cis-Octadecadienoyl oder 9-cis-12-cis-15-cis-Octadecatrienoyl.

Cyclo-$C_3$-$C_7$-alkyl-$C_1$-$C_4$-alkanoyl ist beispielsweise Cyclopentyl-oder Cyclohexylcarbonyl.

Cyclo-$C_3$-$C_7$-alkenyl-$C_1$-$C_4$-alkanoyl ist beispielsweise 1,4-Cyclohexadienylcarbonyl.

Aroyl ist beispielsweise Benzoyl oder 1- oder 2-Naphthoyl, worin der Phenyl- oder Naphthylring durch Nitro, Amino, Halogen, beispielsweise Chlor oder Brom, Hydroxy und/oder $C_1$-$C_4$-Alkoxy, beispielsweise Methoxy, substituiert sein kann, beispielsweise 4-Nitrobenzoyl, 3,5-Dinitrobenzoyl, Anthraniloyl, 2,6-Dichlorbenzoyl, Salicyloyl, Galloyl oder 2-, 3- oder 4-Anisoyl.

$C_1$-$C_7$-Alkoxy-carbonyl ist beispielsweise Methoxy-, Aethoxy-, n-Propoxy-, Isopropoxy-, n-Butoxy-, Isobutoxy- oder tert-Butoxycarbonyl. $C_1$-$C_4$-Alkyl-carbamoyl ist beispielsweise Methyl- oder Aethyl-carbamoyl. Arylcarbamoyl ist beispielsweise Phenylcarbamoyl (Anilinocarbonyl) oder 1- oder 2-Naphthylcarbamoyl, worin der Phenyl- oder Naphthylring durch weitere Substituenten, z.B. Nitro, Amino, Halogen, Hydroxy oder $C_1$-$C_4$-Alkoxy substituiert sein kann, z.B. Anisidinocarbonyl. Di-$C_1$-$C_4$-alkyl-carbamoyl ist beispielsweise Dimethyl- oder Diäthyl-carbamoyl. $C_1$-$C_4$-Alkyl-thiocarbamoyl ist beispielsweise Methyl- oder Aethyl-thiocarbamoyl. Arylthiocarbamoyl ist beispielsweise Phenylthiocarbamoyl. Di-$C_1$-$C_4$-alkyl-thiocarbamoyl ist beispielsweise Dimethyl- oder Diäthyl-thiocarbamoyl. $C_1$-$C_7$- Alkansulfonyl ist beispielsweise Methansulfonyl, Aethansulfonyl oder n-Propansulfonyl. Arylsulfonyl ist beispielsweise Benzolsulfonyl oder p-Toluolsulfonyl.

Salze sind Säureadditionssalze, insbesondere pharmazeutisch verwendbare Säureadditionssalze mit nicht-toxischen und physiologisch gut verträglichen Säuren, beispielsweise Mineralsäuren, wie Chlorwasserstoffsäure, Schwefelsäure oder Phosphorsäure, oder organischen Säuren, wie Niederalkyl-carbonsäuren, beispielsweise Essigsäure, ungesättigte Carbonsäuren, beispielsweise Fumar- oder Maleinsäure, Hydroxycarbonsäuren, beispielsweise Milchsäure, Weinsäure oder Zitronensäure, aromatische Säuren, beispielsweise Salicylsäure, oder Sulfonsäuren, beispielsweise Methansulfonsäure.

Die neuen Saframycin-Derivate der Formel II und ihre Säureadditionssalze haben wertvolle pharmakologische Eigenschaften. Insbesondere haben sie antimikrobielle und anti-Tumor-Eigenschaften und können deshalb therapeutisch in der Form von pharmazeutischen Zubereitungen für die Behandlung von Infektionen, die durch Bakterien verursacht werden, und/oder vorzugsweise für die Behandlung von Tumoren verwendet werden.

Die Saframycin-Derivate der vorliegenden Erfindungen zeigen insbesondere in vitro Zytotoxizität gegenüber Zellkulturen von Mäuse-Leukämie-Zellen L 1210 (NCI). Für die geprüften Verbindungen werden $ID_{50}$-Werte im Bereich von 0,1 bis 0,001 µg/ml gefunden.

Eine in vivo anti-Tumor-Wirkung der Saframycin-Derivate der Formel II wird in verschiedenen Arten von experimentellen Tumormodellen festgestellt, z.B. gegen Mäuse-Leukämie L 1210 in $BDF_1$-Mäusen nach den üblichen Verfahren. Wenn sie während einer Zeitspanne von 9 Tagen 24 Stunden nach der Transplantation des Tumors (100 000 Zellen pro Maus) intraperitoneal verabreicht werden, zeigen mehrere der geprüften Saframycin-Derivate der Formel II eine Zunahme der mittleren Ueberlebens-Tage um bis zu 32 % von Wirkstoff-behan delten Mäusen gegenüber Kontroll-Mäusen, die mit Salzlösung behandelt wurden, bei einer Dosis von zwischen 20 mg und 100 mg pro Maus pro Tag.

Gegen in C57BL/C-Mäuse implantiertes B16-F10 Melanom (200 000 Zellen pro Maus) sind Saframycin-Derivate der Formel II ebenfalls wirksam und führen zu einer Zunahme der mittleren Ueberlebens-Tage von Wirkstoff-behandelten gegenüber von mit Salzlösung behandelten Kontroll-Mäusen von bis zu 35 %. Im weiteren wird eine Wirkung gegen Metastasen von Lewis-Lungenkarzinom in $BDF_1$-Mäusen beobachtet.

Die vorliegende Erfindung betrifft insbesondere Verbindungen der Formel II, worin R Wasserstoff oder Methyl ist.

Bevorzugt sind Verbindungen der Formel II, worin R Wasserstoff oder Methyl, $R_1$ $C_1$-$C_4$-Alkyl, z.B. Methyl, Aethyl, Isopropyl, n-Butyl, Benzyl oder durch Nitro, Amino, Halogen, z.B. Chlor oder Brom, Hydroxy und/oder $C_1$-$C_4$-Alkoxy, z.B. Methoxy, substituiertes Benzyl und $R_2$ Wasserstoff oder $C_1$-$C_4$-Alkyl, z.B. Methyl, Aethyl oder Isopropyl, darstellen, und pharmazeutisch verwendbare Säureadditionssalze dieser Verbindungen.

Gleichermassen bevorzugt sind Verbindungen der Formel II, worin R Wasserstoff oder Methyl, $R_1$ $C_1$-$C_7$-Alkanoyl, z.B. Formyl, Acetyl, Propionyl, n-Butyryl, Isobutyryl, n-Valeryl, Pivaloyl oder n-Hexanoyl, geradkettiges $C_8$-$C_{20}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl oder n-Octadecanoyl, durch Halogen substituiertes $C_2$-$C_7$-Alkanoyl, beispielsweise Trifluoracetyl, Benzoyl, durch Nitro, Amino, Halogen und/oder $C_1$-$C_4$-Alkoxy substituiertes Benzoyl, beispielsweise p-Brombenzoyl, Phenylcarbamoyl, 1-oder 2-Naphthylcarbamoyl oder Phenylthiocarbamoyl und $R_2$ Wasserstoff darstellen, und pharmazeutische verwendbare Säureadditionssalze dieser Verbindungen.

Besonders bevorzugt sind Verbindungen der Formel II, worin R Wasserstoff, $R_1$ $C_1$-$C_4$-Alkyl, z.B. Methyl, Aethyl, Isopropyl, n-Butyl, Benzyl oder durch Nitro, Hydroxy und/oder $C_1$-$C_4$-Alkoxy, z.B. Methoxy, substituiertes Benzyl und $R_2$ Wasserstoff oder $C_1$-$C_4$-Alkyl, z.B. Methyl, Aethyl oder Isopropyl, darstellen oder Verbindungen der Formel II, worin R Wasserstoff oder Methyl, $R_1$ $C_1$-$C_7$-Alkanoyl, z.B. Acetyl, Propionyl, n-Butyryl, Isobutyryl, n-Valeryl, Pivaloyl oder n-Hexanoyl, geradkettiges $C_8$-$C_{18}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl oder n-Octadecanoyl, durch Halogen substituiertes $C_2$-$C_7$-Alkanoyl, z.B. Trifluoracetyl, Benzoyl oder durch Nitro, Amino, Halogen und/oder $C_1$-$C_4$-Alkoxy substituiertes Benzoyl, z.B. p-Brombenzoyl, und $R_2$ Wasserstoff darstellen, und pharmazeutisch verwendbare Säureadditionssalze dieser Verbindungen.

Ganz besonders bevorzugt sind die in den Beispielen beschriebenen Verbindungen.

Verfahren

Die vorliegende Erfindung betrifft ebenso ein Verfahren zur Herstellung der neuen Verbindungen der Formel II. Dieses Verfahren wird nach an sich bekannten Methoden durchgeführt und ist beispielsweise dadurch charakterisiert, dass

a) eine Verbindung der Formel

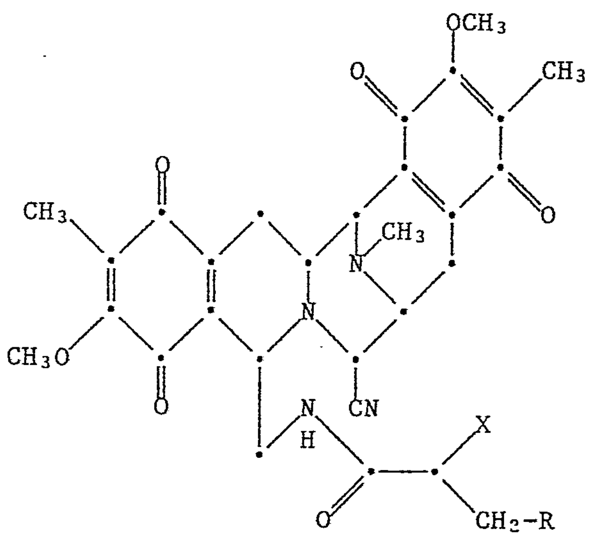

(III),

worin R Wasserstoff oder $C_1$-$C_4$-Alkyl und X eine in eine Gruppe $-NR_1R_2$ überführbare Gruppe darstellen, mit einem Reagens behandelt wird, das die Gruppe $-NR_1R_2$ einführen kann, oder

b) eine Verbindung der Formel

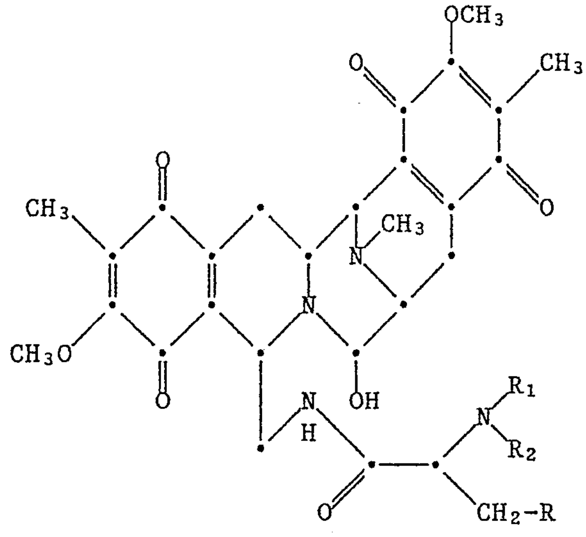

(IV),

worin R, $R_1$ und $R_2$ wie unter Formel II definiert sind, mit Cyanwasserstoff oder einem Salz von Cyanwasserstoff umgesetzt wird, und, wenn erwünscht, eine erhältliche Verbindung der Formel II in ihr Säureadditionssalz oder ein erhältliches Salz in die freie Verbindung oder in ein anderes Säureadditionssalz umgewandelt wird und/oder eine Mischung von Isomeren in die einzelnen Isomeren aufgetrennt wird.

Verfahren a)

Die in eine Gruppe $-NR_1R_2$ überführbare Gruppe X ist beispielsweise die Aminogruppe $-NH_2$, eine Sulfonamid-Gruppe $-NH-SO_2-R'$, eine teilweise substituierte Aminogruppe $-NH-R_1$ oder $-NH-R_2$ oder eine nukleofuge Gruppe. Im entsprechenden Ausgangsmaterial der Formel III hat die die Aminogruppe oder Sulfonamidgruppe X enthaltende Seitenkette bevorzugt die L-Konfiguration.

Die freie Aminogruppe X wird beispielsweise in eine Alkyl-substituierte Aminogruppe durch reduktive Alkylierung überführt, beispielsweise durch Reaktion mit einem $C_1$-$C_7$-Alkylaldehyd, einem Aryl-$C_1$-$C_4$-alkylaldehyd, einem $C_3$-$C_7$-Alkylketon oder mit einem reaktiven funktionellen Derivat dieser Verbindungen und anschliessender Reaktion des Adduktes mit einem Reduktionsmittel.

Ein $C_1$-$C_7$-Alkylaldehyd ist beispielsweise Formaldehyd, Acetaldehyd, Propionaldehyd oder n-Butyraldehyd. Ein Aryl-$C_1$-$C_4$-alkylaldehyd ist beispielsweise Benzaldehyd, p-Nitrobenzaldehyd oder Vanillin. Ein $C_3$-$C_7$-Alkylketon ist beispielsweise Aceton oder Methyläthylketon.

Die Reaktion mit Aldehyden gefolgt von einer Reduktion liefert Verbindungen der Formel II mit einer α-Methylenamino-Einheit $-N-CH_2-$. Die entsprechende Reaktion mit Ketonen ergibt einen α-verzweigten Rest $-N-CH$.

Ein Aequivalent des oben erwähnten Aldehyds oder Ketons ergibt die entsprechenden N-monosubstituirten Derivate, wohingegen zwei Aequivalente oder ein Ueberschuss an Aldehyd die entsprechenden N,N-disubstituierten Derivate ergeben.

Reaktive funktionelle Derivate des oben erwähnten Aldehyds oder Ketons sind beispielsweise Acetale oder Hemiacetale.

Ein geeignetes Reduktionsmittel ist beispielsweise mit einem Edelmetall, wie Palladium, Platin oder Rhodium, oder mit Nickel, wie Raney-Nickel, katalytisch aktivierter Wasserstoff. Vorzugsweise wird eine äquivalente Menge Wasserstoff zugegeben. Andere Reduktionsmittel sind beispielsweise Ameisensäure und Ameisensäurederivate, wie Dimethylformamid oder Ameisensäuremethyl- oder -äthylester (Leuckart-Wallach-Reaktion). Bevorzugt ist ein Hydrid-Reduktionsmittel, z.B. ein Metallkomplex enthaltend mindestens ein bewegliches Hydridion, vorzugsweise ein Aluminium- oder Borhydrid, wie Lithiumaluminiumhydrid, Lithiumtrimethoxyaluminiumhydrid, Natrium-bis(2-methoxyäthoxy)aluminiumhydrid, Natrium- oder Kaliumborhydrid, Natriumcyanoborhydrid oder Natriumtriacetoxy- oder trimethoxyborhydrid. Das bei der Reduktion mit einem Hydrid-Reduktionsmittel erhaltene Reaktionsgemisch wird durch Zugabe von organischen oder anorganischen Säuren, wie verdünnte wässrige Mineralsäure, z.B. wässrige Salzsäure oder Schwefelsäure, solvolysiert.

Die freie Aminogruppe oder eine teilweise substituierte Aminogruppe $-NH-R_1$ oder $-NH-R_2$ kann auch durch Reaktion mit einem Alkylierungsmittel, beispielsweise $C_1$-$C_7$-Alkylhalogenid, z.B. Methyl- oder Aethylchlorid, -bromid oder -jodid, in eine Alkyl-substituierte Aminogruppe $-NR_1R_2$ umgewandelt werden. Für die Herstellung einer Verbindung der Formel II, worin $R_1$ und $R_2$ $C_1$-$C_7$-Alkyl bedeuten, werden vorzugsweise zwei Aequivalente des $C_1$-$C_7$-Alkylhalogenids zugegeben. Diese Reaktion wird in Gegenwart einer Base, vorzugsweise eines tertiären Amins, wie Triäthylamin oder Pyridin, oder eines Amidins, wie 1,8-Diazabicyclo[5.4.0]undec-7-en, durchgeführt.

Die Alkylierungsreaktionen werden vorzugsweise in einem inerten, vorzugsweise polaren wasserfreien Lösungsmittel durchgeführt, z.B. in einem dipolar aprotischen Lösungsmittel, oder in Lösungsmittelgemischen in einem Temperaturbereich von ungefähr -40°C bis zum Siedepunkt der Reaktionsmischung, vorzugsweise von ungefähr 0°C bis 50°C, wenn erwünscht in einer inerten Gas-Atmosphäre, z.B. unter Stickstoff.

Eine freie Aminogruppe X oder eine teilweise substituierte Aminogruppe $-NHR_2$ wird in eine acylierte Aminogruppe $-NR_1R_2$ mit einer Säure der Formel $R_1$-OH oder einem reaktiven funktionellen Derivat dieser Säure umgewandelt.

Wenn eine freie Carbonsäure oder Sulfonsäure für die Acylierung verwendet wird, wird die Reaktion üblicherweise in Gegenwart eines geeigneten Kondensationsmittels durchgeführt, z.B. eines Carbodiimids, beispielsweise Diäthyl-, Dipropyl-, Dicyclohexyl- oder N-Aethyl-N'-3-dimethylaminopropyl-carbodiimid.

Die Kondensationsreaktion wird vorzugsweise in flüssiger Phase in Gegenwart eines Lösungsmittels durchgeführt, gegebenenfalls unter Kühlung oder Erwärmung, beispielsweise in einem Temperaturbereich von ungefähr -40°C bis zum Siedepunkt der Reaktionslösung, vorzugsweise zwischen Raumtemperatur und der Siedetemperatur der Reaktionsmischung, und gegebenenfalls in einer inerten Gas-Atmosphäre, beispielsweise Stickstoff-Atmosphäre.

Ein reaktives funktionelles Derivat der Carbonsäure oder Sulfonsäure ist ein entsprechendes Anhydrid, beispielsweise ein symmetrisches Anhydrid, z.B. Essigsäureanhydrid, oder ein gemischtes Anhydrid der Säure $R_1$-OH mit einer anorganischen oder organischen Säure, z.B. einer Halogenwasserstoffsäure, z.B. Salzsäure oder Bromwasserstoffsäure, wie eines Carbonsäurechlorids oder -bromids oder eines Sulfonsäurechlorids oder -bromids, oder mit einem Halbester der Kohlensäure, z.B. dem Aethyl- oder Isobutyl-halbester der Kohlensäure.

Ein weiteres reaktives funktionelles Derivat einer Carbonsäure $R_1$-OH ist beispielsweise ein aktivierter Ester, z.B. ein N-Hydroxyester, wie der Ester mit N-Hydroxypiperidin, N-Hydroxysuccinimid oder N-Hydroxyphthalimid.

Ein reaktives funktionelles Derivat einer Säure $R_1$-OH ist im weiteren beispielsweise ein Isocyanat oder Thioisocyanat, beispielsweise Phenylisocyanat oder Phenylisothiocyanat, ein N-$C_1$-$C_4$-Alkyl-O-$C_2$-$C_4$-alkylurethan, z.B. N-Methyl-O-äthylurethan, ein N-$C_1$-$C_4$-Alkyl-O-$C_2$-$C_4$-alkylthiocarbamat, z.B. N-Methyl-O-äthylthiocarbamat, ein N,N-Di-$C_1$-$C_4$-alkyl-O-$C_2$-$C_4$-alkylurethan oder das entsprechende N,N-Di-$C_1$-$C_4$-alkyl-O-$C_2$-$C_4$-alkylthiocarbamat.

Während der Durchführung des Acylierungsverfahrens sind funktionelle Gruppen in $R_1$, beispielsweise Hydroxygruppen, vorzugsweise in geschützter Form. Schutzgruppen, ihre Einführung und ihre anschliessende Abspaltung sind in den folgenden Literaturstellen beschrieben: J.F.W. McOmie, "Protective Groups in Organic Chemistry", Plenum Press, London, New York, 1973; T.W. Greene, "Protective Groups in Organic Synthesis", Wiley, New York, 1981; "The Peptides", Bd. 3, Herausgeber E. Gross und J. Meienhofer, Academic Press, London, New York, 1981 und Houben-Weyl, "Methoden der Organischen Chemie", Band 15/1, Georg Thieme Verlag, Stuttgart, 1974.

Geeignete Hydroxy-Schutzgruppen sind beispielsweise die Acylgruppe eines Kohlensäurehalbesters, beispielsweise 2,2,2-Trichloräthoxycarbonyl, Allyloxycarbonyl, Benzyloxycarbonyl oder p-Nitrobenzyloxycarbonyl, oder Triniederalkylsilyl, beispielsweise Trimethylsilyl oder Dimethyl-tert-butylsilyl.

Die obengenannten Acylierungsreaktionen mit einem reaktiven funktionellen Derivat werden vorzugsweise in Gegenwart eines geeigneten Säureakzeptors durchgeführt, beispielsweise einer geeigneten organischen Base, z.B. eines Amins, z.B. eines tertiären Amins wie Triniederalkylamin, z.B. Trimethylamin oder Triäthylamin, eines cyclischen tertiären Amins wie N-Methylmorpholin, eines bicyclischen Amidins, z.B. eines Diazabicycloalkens wie 1,5-Diazabicyclo[4.3.0.]non-5-en oder 1,8-Diazabicyclo[5.4.0]undec-7-en (DBU), oder beispielsweise einer Base der Pyridintyps, z.B. Pyridin. Ein geeigneter Säureakzeptor ist ebenfalls eine anorganische Base, beispielsweise ein Alkalimetall-hyhdroxid oder ein Erdalkalimetall-hydroxid, z.B. Natrium-, Kalium- oder Calcium-hydroxid.

Die Acylierungsreaktionen mit reaktiven funktionellen Derivaten von $R_1$-OH werden vorzugsweise in einem inerten, vorzugsweise wasserfreien Lösungsmittel oder in Lösungsmittelgemischen durchgeführt, wenn erwünscht bei tiefen oder erhöhten Temperaturen, z.B. im Temperaturbereich von ungefähr -40°C bis zum Siedepunkt der Reaktionsmischung, vorzugsweise von Raumtemperatur bis zum Siedepunkt der Reaktionsmischung, und gegebenenfalls in einer Inertgas-Atmosphäre, z.B. unter Stickstoff.

Die Sulfonamidgruppe $-NH-SO_2-R'$ einen aliphatischen oder aromatischen Rest, z.B. $C_1$-$C_4$-Alkyl, z.B. Methyl, oder Aryl, z.B. Phenyl oder Tolyl, bedeutet, wird in eine Gruppe $-NH-R_1$ mit einem Halogenid der Formel $R_1$-Hal (Hal = Cl, Br, I), z.B. Methylbromid oder Methyljodid, Benzylchlorid oder Acetylchlorid, in Gegenwart einer anorganischen oder organischen Base, z.B. verdünnter wässriger Kalium- oder Natronlauge oder eines tertiären aliphatischen oder aromatischen Amins wie Triäthylamin, Pyridin oder 1,8-Diazabicyclo[5.4.0]undec-7-en, umgewandelt. Zu diesem Zweck wird das Sulfonamid vorzugsweise in ein Salz umgewandelt, z.B. mit einem Ueberschuss einer oben erwähnten Base oder durch Reaktion mit Alkalimetallamiden, Alkalimetallhydriden oder Alkalimetallalkyl-Verbindungen. Das Alkalimetall ist vorzugsweise Lithium oder Natrium. Geeignete Reagenzien sind beispielsweise Natrium- oder Lithiumamid, Lithium-bis(trimethylsilyl)amid, N,O-bis(trimethylsilyl)acetamid, Natrium-oder Lithiumhydrid oder vorzugsweise Lithium-diisopropylamid oder n-Butyllithium. Die Reaktion wird in einem inerten, vorzugsweise aprotischen

Lösungsmittel in einem Temperaturbereich von ungefähr -80° C bis zum Siedepunkt der Reaktionsmischung, z.B. zwischen -30° C und Raumtemperatur, durchgeführt.

Eine nukleofuge Gruppe X ist vorzugsweise verestertes Hydroxy, wie Halogen, z.B. Chlor, Brom oder Jod, Niederalkanoyloxy, wie Acetoxy, oder Sulfonyloxy, wie p-Toluolsulfonyloxy, Benzolsulfonyloxy oder Mesyloxy. Die nukleofuge Gruppe wird durch Reaktion mit einem Amin der Formel $HNR_1R_2$ durch eine Gruppe $-NR_1R_2$ ausgetauscht.

Vorzugsweise wird als Ausgangsmaterial der Formel III eine Verbindung gewählt, worin das Kohlenstoffatom, das die nukleofuge Gruppe X trägt, eine der Konfiguration im entsprechenden, die Aminogruppe tragenden Kohlenstoffatom im Endprodukt der Formel II entgegengesetzte Konfiguration aufweist.

Das Amin $HNR_1R_2$ wird in wenigstens äquivalenten Mengen, vorzugsweise in einem Ueberschuss von 2-5 Aequivalenten, zugegeben. Die Reaktion mit nur einem Aequivalent des Amins erfordert die Gegenwart einer anderen Base, z.B. eines tertiären aliphatischen Amins, wie Triäthylamin, Pyridin oder 1,8-Diazabicyclo[5.4.0]undec-7-en. Wenn erwünscht kann auch eine andere Base zugegeben werden, wenn mehr als ein Aequivalent des Amins $NHR_1R_2$ mit der Verbindung der Formel III umgesetzt wird.

Die Reaktion wird in Lösungsmitteln durchgeführt, die eine bimolekulare Substitutionsreaktion vom "$S_N2$"-Typ erleichtern, beispielsweise dipolar aprotische Lösungsmittel. Die Reaktionstemperatur liegt zwischen -20°C und dem Siedepunkt der Reaktionsmischung, vorzugsweise bei Temperaturen über Raumtemperatur bis zum Siedepunkt der Reaktionsmischung. Wenn nötig wird die Reaktion in einer Inertgas-Atmosphäre, z.B. unter Stickstoff oder Argon, durchgeführt.

Verfahren b)

Salze von Cyanwasserstoff sind beispielsweise Ammoniumcyanid, Alkalimetall- oder Erdalkalimetall-cyanide, z.B. Natrium- oder Kaliumcyanid, oder ein Uebergangsmetall-cyanid wie Kupfercyanid. Vorzugsweise wird Natrium- oder Kaliumcyanid in situ in einem molaren Ueberschuss zum die Verbindung der Formel IV enthaltenden Kulturfiltrat zugegeben. Die Herstellung des eine Verbindung der Formel IV enthaltenden Kulturfiltrats ist nachfolgend unter "Ausgangsmaterialien" beschrieben. Die Reaktionstemperatur liegt zwischen -40°C und der Siedetemperatur der Reaktionsmischung, vorzugsweise um Raumtemperatur.

Die nach der vorliegenden Erfindung erhältlichen Verbindungen können aus der Reaktionsmischung unter schwach alkalischen Bedingungen mit einem organischen Lösungsmittel, das nicht mit Wasser mischbar ist, beispielsweise Methylenchlorid, extrahiert werden. Das Lösungsmittel wird beispielsweise durch Destillation unter vermindertem Druck entfernt. Der so erhaltene Rückstand kann durch das folgende extraktive Verfahren gereinigt werden: Er wird in einem organischen Lösungsmittel, z.B. Essigsäureäthylester, gelöst und die organische Lösung beispielsweise durch Zugabe einer Lösung von wässriger Essigsäure angesäuert. Die das Produkt enthaltende saure wässrige Phase wird schwach alkalisch gestellt, beispielsweise durch Zugabe von wässrigem Ammoniak oder einer wässrigen Natriumcarbonat-Lösung. Das Produkt wird wieder durch ein organisches Lösungsmittel, beispielsweise Essigsäureäthylester, extrahiert. Abhängig vom gewünschten Reinheitsgrad werden die Extraktionsverfahren wiederholt. Das Lösungsmittel wird aus der organischen Lösung abdestilliert, der Rückstand getrocknet und das erhältliche Produkt, wenn erwünscht, durch chromatographische Methoden, z.B. Kieselgel-Säulenchromatographie, weiter gereinigt.

Die Abspaltung von Schutzgruppen, z.B. Hydroxy-Schutzgruppen, ist in den oben erwähnten Literatur-Referenzen beschrieben und wird beispielsweise durch Hydrolyse mit Säuren, wie Trifluoressigsäure, oder durch reduktive Methoden, wie Reaktion mit naszierendem Wasserstoff, bewerkstelligt.

Säureadditionssalze werden nach den üblichen Methoden erhalten, z.B. durch Behandlung mit der entsprechenden Säure oder mit einem geeigneten Anionenaustauscher. Ein Säureadditionssalz wird in ein anderes Säureadditionssalz nach den üblichen Methoden umgewandelt, z.B. durch Behandlung mit einem Ueberschuss der entsprechenden Säure oder mit einem geeigneten Anionenaustauscher. Die freie Verbindung wird aus einem Säureadditionssalz durch Behandlung mit einer Base erhalten.

Die Trennung von durch das Verfahren der Erfindung erhältlichen Gemischen von Diastereoisomeren in die einzelnen Diastereomeren wird nach an sich bekannten Methoden erreicht, beispielsweise durch physikalische oder chemische Methoden, beispielsweise durch fraktionierte Kristallisation oder durch chromatographische Methoden, z.B. Flüssig-Chromatographie.

Die Trennung von durch das Verfahren der Erfindung erhältlichen Racematen in optisch reine Antipoden wird durch an sich bekannte Methoden erreicht, beispielsweise durch fraktionierte Kristallisation von diastereomeren Salzen, die mit chiralen Säuren hergestellt werden, oder durch Chromatographie an optisch aktiven Adsorptionsflächen. Die Racemate können auch in optisch aktiven Lösungsmitteln gelöst und das weniger lösliche optische Antipode aus der so erhaltenen Lösung kristallisiert werden. Weiter kann auch die unterschiedliche Reaktivität der optischen Antipoden gegenüber biologischem Material, wie Mikroorganismen oder isolierten Enzymen, ausgenutzt werden, oder die Racemate werden gelöst und ein optisches Isomer durch Animpfen der Lösung mit einer kleinen Menge eines optisch aktiven Produkts, das nach einer der oben erwähnten Methoden erhalten worden ist, auskristallisiert.

Das Verfahren umfasst auch diejenigen Ausführungsformen, bei welchen als Zwischenprodukte erhältliche Verbindungen als Ausgangsmaterialien verwendet werden und die verbleibenden Verfahrensschritte damit durchgeführt werden oder man das Verfahren auf irgendeiner Stufe unterbricht. Ferner können Ausgangsmaterialien in der Form von Derivaten verwendet werden oder während der Reaktion gebildet werden.

Vorzugsweise werden diejenigen Ausgangsmaterialien verwendet und solche Reaktionsbedingungen gewählt, bei denen die oben beschriebenen bevorzugten Verbindungen gebildet werden.

Ausgangsmaterialien

Die im Verfahren zur Herstellung der Verbindung der Formel II verwendeten Ausgangsmaterialien sind bekannt oder, wenn sie neu sind, können nach bekannten Methoden erhalten.

Verbindungen der Formel III, worin X $NH_2$ bedeutet, z.B. Verbindungen der Formel I, sind bekannt und können nach den Methoden, wie sie in der oben erwähnten Publikation: "Directed Biosynthesis of New Saframycin Derivatives with Resting Cells of Streptomyces lavendulae" von Tadashi Arai et al. beschrieben sind, hergestellt werden.

Verbindungen der Formel III, worin X die Sulfonamidgrupe $-NH-SO_2-R'$ darstellt, werden nach bekannten Methoden hergestellt, beispielsweise indem man eine Verbindung der Formel I mit einem entsprechenden geeigneten Sulfonsäurederivat, z.B. Mesylchlorid oder p-Toluolsulfonylchlorid, wie oben für die Acylierung von Verbindungen der Formel III beschrieben, umsetzt.

Verbindungen der Formel IV können durch Reaktion einer wässrigen Suspension der Biomasse eines Stammes des Genus Streptomyces lavendulae, der Saframycin A herstellen kann, mit einem substituierten Aminosäure-Derivat der Formel

$$\begin{array}{l} HOOC-CH-CH_2-R \\ \qquad\quad | \\ \qquad\;\; NR_1R_2 \end{array} \qquad (V),$$

worin R, $R_1$ und $R_2$ wie oben unter Formel II definiert sind, in Gegenwart von Glycin, L-Tyrosin und L-Methionin hergestellt werden.

Ein Stamm des Genus Streptomyces, der Saframycin Z herstellen kann, ist vorzugsweise der Stamm Streptomyces lavendulae Nr. 314 oder eine Mutante, die von diesem Mikroorganismus abgeleitet ist und ebenfalls Saframycin A produziert. Die Isolierung und Reinigung von Streptomyces lavendulae Nr. 314 aus einer Bodenprobe der Gegend von Kyoto sowie die taxonomische Klassierung dieses Stammes ist in der US-Patentschrift Nr. 4,248,863 beschrieben.

Der Mikroorganismus wird unter aeroben Bedingungen, beispielsweise in einer Atmosphäre enthaltend Sauerstoff, mit Sauerstoff angereicherte Luft oder Luft, und unter Schütteln oder Rühren in Schüttelflaschen oder Fermentern kultiviert. Bevorzugt sind ständig gerührte Suspensions-Kulturen. Die Kultivierung wird in einem Temperaturbereich von ungefähr 25°C bis ungefähr 35°C, vorzugsweise von ungefähr 27°C bis ungefähr 30°C, durchgeführt.

Kultiviert werden kann in Partien, beispielsweise durch einzelne oder mehrfache Zugabe von Nährlösung, oder kontinuierlich durch wiederholte Zugabe von Nährlösung.

Vorzugsweise wird in verschiedenen Stufen kultiviert, indem man eine oder mehrere Vorkulturen vor der oben erwähnten Hauptkultur herstellt. Die erste Kultur wird in einem synthetischen Nährmedium hergestellt, vorzugsweise in Krainsky-Agar-Schrägkultur, [Natur. Centr. Bakteriol. Parasitenk., Abt. II, 41, 649-688 (1914)] enthaltend Bakto-Agar (Difco), Glukose, Asparagin, $KH_2PO_4$ und destilliertes Wasser. Diese Kultur wird in eine andere, Kohlenstoff- und Stickstoff-Quellen sowie essentielle Spurenelemente enthaltende Kultur überführt.

Kohlenstoff-Quellen sind Kohlenhydrate wie D-Glukose, Maltose, D-Fructose, L-Arabinose oder Rohrzucker oder Salze von organischen Säuren wie Natriumacetat, Natriumcitrat oder Natriumsuccinat.

Stickstoff-Quellen sind Fleichshextrakte, Polypepton, Trypton, Gluten, Baumwollsamenöl, Sojabohnenmehl, flüssige Maisstärke, getrocknete Hefe, Hefeextrakte, Harnstoff, Ammoniumsalze, beispielsweise Ammoniumchlorid oder Ammoniumsulfat, oder Nitrate, beispielsweise Kalium- oder Ammoniumnitrat.

Essentielle Spurenelemente werden in Form von anorganischen Salzen zugegeben. Solche Salze sind beispielsweise wasserlösliche Halogenide, beispielsweise Chloride, Carbonate, Sulfate oder Phosphate von Alkalimetallen, beispielsweise Natrium oder Kalium, Erdalkalimetallen, beispielsweise Calcium oder Magnesium, oder Uebergangsmetallen, beispielsweise Eisen, Mangan, Molybdän, Kupfer oder Zink.

Falls Stickstoff-Quellen aus Fleisch oder Pflanzen verwendet werden, wie Fleischextrakte, Proteinhydrolysate oder Pepton etc., ist die Zugabe von essentiellen Spurenelementen nicht unbedingt nötig, weil diese Elemente schon in den erwähnten Stickstoff-Quellen vorhanden sein können.

Der Verlauf der Fermentation kann analytisch an gezogenen Proben verfolgt werden, beispielsweise durch Messen der optischen Dichte, welche ein Mass für das Wachstum eines einzelnen Stammes ist, sowie auch durch gravimetrische Analyse auf der Basis des Trockengewichts der gebildeten Biomasse.

In Fall dass während der Fermentation Schäume gebildet werden, wird ein Antischäummittel, wie Silikon, pflanzliches Oel (Sojabohnenöl) oder Mineralöl-Antischäummittel, wie Polyoxyalkylen, Adecanol® oder ähnliches zugegeben.

Biomasse oder Mycel wird beispielsweise durch Fermentation der oben genannten Mikroorganismen gebildet und kann von der wässrigen Phase durch klassische Trennmethoden, beispielsweise Filtration, Zentrifugation oder Sedimentation, abgetrennt werden.

Die aus dem Kulturmedium gewonnene Biomasse oder Mycel wird anschliessend wieder in wässriger

Phase, die den Mikroorganismus am Leben erhalten kann, suspendiert, vorzugsweise in gepufferter wässriger isotonischer Natriumchlorid-Lösung, z.B. in 0,1 M 2-(N-morpholino)-äthansulfonsäure-monohydrat-Puffer.

Vorzugsweise werden ungefähr 5 g der Biomasse in 100 g Wasser suspendiert. Zu dieser Suspension werden die Aminosäuren L-Methionin, L-Tyrosin, Glycin und die substituierten Aminosäure-Derivate der Formel V oder ein Peptid aus diesen Aminosäuren zugegeben und die Reaktion wie oben erwähnt belüftet, geschüttelt oder gerührt. Vorzugsweise wird ein substituiertes Aminosäure-Derivat der Formel V mit der L-Konfiguration zugegeben.

Die Biomasse wird nach Reaktion mit den oben erwähnten Aminosäuren und/oder Peptiden von der wässrigen Phase durch klassische Trennmethoden abgetrennt, beispielsweise durch Filtration. Zum Kulturfiltrat wird ein Ueberschuss eines Cyanids, z.B. eines Alkalimetall-cyanids, beispielsweise Natrium- oder Kalium-cyanid, zugegeben.

Verbindungen der Formel III, worin X eine nukleofuge Gruppe darstellt, werden durch eine Abwandlung des Verfahrens zur Herstellung von Ausgangsmaterial der Formel IV erhalten. Die Suspension der Biomassse eines Stammes, der Saframycin A herstellen kann, z.B. Streptomyces lavendulae Nr. 314, wird dabei mit der Säure der Formel

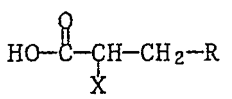
(VI),

worin R und X wie oben definiert sind, beispielsweise mit 2-Chlorpropionsäure oder Milchsäure, gegebenenfalls in Gegenwart von Glycin, L-Tyrosin und L-Methionin oder mit einem Acyl-peptid aus diesen Aminosäuren und der Säure der Formel VI, umgesetzt. Verbindungen der Formel III, worin X Hydroxy ist, können durch Umwandlung in die entsprechenden p-Toluolsulfonyloxy- oder Mesyloxy-Derivate aktiviert werden.

Verbindungen der Formel V sind bekannt und werden nach an sich bekannten Methoden hergestellt, beispielsweise durch Reaktion der α-substituierten Säure der Formel VI, worin R und X die unter Formel III genannten Bedeutungen haben, mit einem Reagens, das die Gruppe $-NR_1R_2$ einführen kann. Dieses Verfahren wird analog zum Verfahren a) durchgeführt.

Verwendung

Die Erfindung betrifft ebenso die Verwendung der neuen Verbindungen der Formel II und ihrer pharmazeutisch annehmbaren Säureadditionssalze als pharmakologisch wirksame Verbindungen in der Behandlung von Warmblütern, beispielsweise Menschen, vorzugsweise in der Form von pharmazeutischen Zusammensetzungen. Die Dosis der wirksamen Verbindungen hängt ab von der Spezies, vom Gewicht, Alter und dem individuellen Zustand, und ebenso von der Art der Anwendung. Beispielsweise wird eine Tagedosis im Bereich von 5 mg bis 100 mg, vorzugsweise 10 mg bis 70 mg der aktiven Verbindung pro ungefähr 70 kg Körpergewicht verabreicht.

Die neuen Saframycin-Derivate können ebenfalls für tierärztliche Zwecke verwendet werden.

Die pharmazeutischen Zubereitungen der vorliegenden Erfindung sind für enterale oder parenterale Verabreichung brauchbar und enthalten die pharmakologisch wirksame Verbindung der Formel II oder ein pharmazeutisch verwendbares Säureadditionssalz davon, gegebenenfalls weitere pharmazeutsich wirksame Verbindungen, und einen geeigneten pharmazeutisch verwendbaren Träger. Die pharmazeutischen Zubereitungen werden nach an sich bekannten Methoden hergestellt, beispielsweise durch klassische Lösungs- oder Lyophilisierungs-Verfahren, und enthalten von ungefähr 0,1 % bis 100 %, insbesondere von ungefähr 1 % bis ungefähr 50 %, im Falle von Lyophilisaten bis zu 100 %, der aktiven Verbindung.

Die pharmazeutischen Zubereitungen der vorliegenden Erfindung werden vorzugsweise in der Form von parenteralen, beispielsweise intravenös oder intraperitoneal anwendbaren Zubereitungen oder Infusions-Lösungen verabreicht. Solche Zubereitungen oder Lösungen sind vorzugsweise isotonische wässrige Lösungen oder Suspensionen, z.B. ölige Injektions-Suspensionen. Diese Lösungen oder Suspensionen können vor Gebrauch hergestellt werden, beispielsweise aus lyophilisierten Zubereitungen, die die wirksamen Verbindungen selbst oder in Verbindung mit einem Träger, beispielsweise Mannitol, enthalten. Die pharmazeutischen Zubereitungen werden sterilisiert und/oder können Hilfsstoffe enthalten, beispielsweise Konservierungsmittel, Stabilisatoren, Netzmittel, Emulgatoren, Löslichkeitsvermittler, Salze zur Regulierung des osmotischen Druckes und/oder Puffer. Wässrige Injektions-Suspensionen können durch Zumischung von Stoffen, welche die Viskosität erhöhen, hergestellt werden, beispielsweise mit Natrium-carboxymethylcellulose, Sorbit und/oder Dextran, und gegebenenfalls mit Stabilisiermittel.

Zur parenteralen Verabreichung geeignet sind insbesondere dispergierte Liposomen enthaltend Verbindungen der Formel II. Die dispergierten Liposomen werden aus mindestens zwei Fettkomponenten gebildet, beispielsweise Phosphatidylserin und Phosphatidyläthanolamin (Kephalin) oder Phosphatidylserin und Phosphatidylcholin (Lecithin), welche die aktive Verbindung der Formel II einschliessen.

Geeignet zur enteralen, beispielsweise oralen, Verabreichung sind pharmazeutische Zusammensetzungen, die von ungefähr 10 % bis ungefähr 90 %, insbesondere von 20 % bis 75 %, der wirksamen Verbindung selbst in Verbindung mit einem pharmazeutisch verwendbaren Träger enthalten. Diese Zubereitungen werden

in Form von Dosiereinheiten, wie Dragées, Tabletten, Kapseln, Suppositorien oder Ampullen hergestellt. Sie werden nach an sich bekannten Methoden zubereitet, beispielsweise mit Hilfe konventioneller Misch-, Granulier-, Ueberzieh-, Lösungs- oder Lyophilisierungs-Verfahren.

Geeignete Träger für die Herstellung von Tabletten und/oder Dragées sind insbesondere Füllmaterialien wie Zucker, beispielsweise Laktose, Saccharose, Mannit oder Sorbit, Cellulose-Zubereitungen und/oder Kalziumphosphate, beispielsweise Trikalziumphosphat oder Kalziumbiphosphat, ebenso Bindemittel, wie Stärkepasten unter Verwendung von beispielsweise Mais-, Weizen-, Reis- oder Kartoffel-Stärke, Gelatine, Tragakanth, Methylcellulose, Hydroxypropylmethylcellulose, Natrium-carboxymethylcellulose and/oder Polyvinylpyrrolidon, und/oder, wenn erwünscht, Sprengmittel, beispielsweise die oben genannten Stärken, ebenfalls Carboxymethyl-Stärke, quervernetztes Polyvinylpyrrolidon, Agar, Alginsäure oder Salze davon, wie Natriumalginat. Zusatzstoffe sind insbesondere Fliessreguliermittel und Schmiermittel, beispielsweise Kieselsäure, Talg, Stearinsäure oder Salze davon, wie Magnesium- oder Kalziumstearat, und/oder Polyäthylenglykol. Dragées-Kerne werden mit geeigneten Ueberzügen, die gegebenenfalls gegenüber Magensaft resistent sind, versehen. Unter anderem werden Zuckerlösungen, welche gegebenenfalls Gummi arabicum, Talg, Polyvinylpyrrolidon, Polyäthylenglykol und/oder Titandioxid enthalten, Lackierlösungen in geeigneten organischen Lösungsmitteln oder Lösungsmittelgemischen oder, für die Herstellung von Magensaft-resistenten Ueberzügen, Lösungen von geeigneten Cellulose-Zubereitungen, wie Acetylcellulose-phthalat oder Hydroxypropylmethylcellulose-phthalat, verwendet. Zu den Tabletten oder Dragée-Ueberzügen können Farbstoffe oder Pigmente zugegeben werden, beispielsweise zur Identifizierung oder zur Bezeichnung von unterschiedlichen Mengen des aktiven Wirkstoffs.

Weitere pharmazeutische Zubereitungen für orale Anwendung sind trocken-gefüllte Kapseln aus Gelatine und geschlossene weiche Kapseln aus Gelatine und einem Weichmacher, wie Glycerin oder Sorbit.

Die nachfolgenden Beispiele dienen zur Illustration der Erfindung, schränken aber deren Umfang in keiner Weise ein. Temperaturen werden in Grad Celsius angegeben.

Beispiel 1: N,N-Diäthyl-Saframycin $Y_3$

280 mg (0,5 mMol) Saframycin $Y_3$ werden in 10 ml Acetonitril gelöst und mit 110 mg (2,5 mMol) Acetaldehyd versetzt. Nach ein paar Minuten werden 13 mg (0,2 mMol) Natriumcyanoborhydrid zugegeben. Die Reaktionsmischung wird eine Stunde bei Raumtemperatur gerührt, dann in 200 ml Essigsäureäthylester gegossen und zweimal mit 1 N Salzsäure extrahiert. Die wässrige Phase wird mit wässriger Ammoniak-Lösung leicht basisch gestellt (pH 9). Die organische Phase wird mit Wasser gewaschen und über wasserfreiem Natriumsulfat getrocknet. Das Lösungsmittel wird abgedampft und der Rückstand durch Säulenchromatographie an Kieselgel mit Benzol/Essigsäureäthylester (1:1, 1:2, 1:4) gereinigt. Das Produkt wird durch präparative Kieselgel-Dünnschichtchromatographie mit Chloroform/Methanol (20:1) als Laufmittel gereinigt. Rf: 0,41 (Essigsäureäthylester/Benzol 4:1), 0,60 (Chloroform/Aethanol 20:1), Smp. 108-112°.

Beispiel 2: Analog Beispiel 1 werden
N,N-Di-n-propyl-Saframycin $Y_3$ (Rf: 0,62, 0.70; Smp. 97-99°) und
N,N-Di-n-butyl-Saframycin $Y_3$ (Rf: 0,64, 0,73; Smp. 73-75°)
durch Reaktion von 280 mg Saframycin $Y_3$ (0,5 mMol) mit 2,5 mMol des entsprechenden Aldehyds und Reduktion mit 13 mg (0,2 mMol) Natriumcyanoborhydrid hergestellt. Rf-Werte sind für Essigsäureäthylester/Benzol 4:1 und Chloroform/Aethanol 20:1 angegeben.

Beispiel 3: Analog Beispiel 1 werden
N-Propyl-Saframycin $Y_3$ (Rf: 0,26, 0,49; Smp. 116-119°),
N-Isopropyl-Saframycin $Y_3$ (Rf: 0,18, 0,43; Smp. 114-118°),
N-n-Butyl-Saframycin $Y_3$ (Rf: 0,30 0,51; Smp. 101-103°),
N-Benzyl-Saframycin $Y_3$ (Rf: 0,51, 0,65; Smp. 110-112°),
N-p-Nitrobenzyl-Saframycin $Y_3$ (Rf: 0,42, 0,61; Smp. 178-181°),
N-Vanillyl-Saframycin $Y_3$ (Rf: 0,36, 0,47; Smp. 127-131°)
durch Reaktion von 280 mg Saframycin $Y_3$ (0,5 mMol) mit 0,5 mMol des entsprechenden Aldehyds oder Ketons und Reduktion mit 13 mg (0,2 mMol) Natriumcyanoborhydrid hergestelt. Rf-Werte werden für Essigsäureäthylester/Benzol 4:1 und Chloroform/Aethanol 20:1 angegeben.

Beispiel 4: N-Acetyl-Saframcyin $Y_3$

280 mg (0,5 mMol) Saframycin $Y_3$ werden in 40 ml wasserfreiem Benzol gelöst. 204,2 mg (2,0 mMol) Essigsäureanhydrid und 253 mg (2,5 mMol) Triäthylamin werden zur Lösung zugegeben. Die Reaktionsmischung wird 3 Stunden bei Raumtemperatur gerührt, dann in 200 ml Essigsäureäthylester gegossen. Diese Mischung wird mit 1 N wässriger Natriumcarbonat-Lösung und mit Wasser gewaschen. Die organische Phase wird mit wasserfreiem Natriumsulfat getrocknet und das Lösungsmittel abgedampft. Der Rückstand wird durch Säulenchromatographie an Kieselgel 60 (Merck Deutschland) mit Benzol und Essigsäureäthylester als Laufmittel (10:1, 4:1, 2:1) gereinigt. Das Produkt wird weiter durch präparative Kieselgel-Dünnschichtchromatographie (TLC) gereinigt. Rf: 0,21, 0,13 (Benzol/Essigsäureäthylester 1:2, 1:1); Smp. 131-135°.

Beispiel 5: N-Trifluoracetyl-Saframycin $Y_3$

Analog Beispiel 4 wird die Titelverbindung durch Reaktion von 280 mg (0,5 mMol) Saframycin $Y_3$ mit 420,1 mg (2,0 mMol) Trifluoressigsäureanhydrid in Gegenwart von 253 mg (2,5 mMol) Triäthylamin erhalten. Rf: 0,43,

0,39 (Benzol/Essigsäureäthylester 1:2, 1:1); Smp. 131-137°.

Beispiel 6: N-Propionyl-Saframycin $Y_3$

280 mg (0,5 mMol) Saframycin $Y_3$ werden in 40 ml wasserfreiem Benzol gelöst. 160 mg (2,0 mMol) Propionylchlorid und 253 mg (2,5 mMol) Triäthylamin werden zur Lösung zugegeben. Das Reaktionsgemisch wird 3 Stunden bei Raumtemperatur gerührt, dann in 200 ml Essigsäureäthylester gegossen. Diese Mischung wird mit 1 N wässriger Natriumcarbonat-Lösung und mit Wasser gewaschen. Die organische Phase wird mit wasserfreiem Natriumsulfat getrocknet und das Lösungsmittel abgedampft. Der Rückstand wird durch Säulenchromatographie an Kieselgel 60 (Merck Deutschland) mit Benzol und Essigsäureäthylester als Laufmittel (10:1, 4:1, 2:1) gereinigt. Das Produkt wird weiter durch präparative Kieselgel-Dünnschichtchromatographie (TLC) gereinigt. Rf: 0,26, 0,19 (Benzol/Essigsäureäthylester 1:2, 1:1); Smp. 111-114°.

Beispiel 7: Analog Beispiel 6 werden
N-Pivaloyl-Saframycin $Y_3$ (Rf: 0,37, 0,29; Smp. 194-197° Zers.);
N-Butyryl-Saframycin $Y_3$ (Rf: 0,30, 0,24; Smp. 118-121°);
N-Valeryl-Saframycin $Y_3$ (Rf: 0,34, 0,27; Smp. 108-111°);
N-n-Hexanoyl-Saframycin $Y_3$ (Rf: 0,39, 0,27; Smp. 109-112°);
N-n-Octanoyl-Saframycin $Y_3$ (Rf: 0,42, 0,31; Smp. 97-100°);
N-n-Decanoyl-Saframycin $Y_3$ (Rf: 0,43, 0,33; Smp. 97- 99°);
N-n-Dodecanoyl-Saframycin $Y_3$ (Rf: 0,43, 0,35; Smp. 89- 92°);
N-n-Tetradecanoyl-Saframycin $Y_3$ (Rf: 0,43, 0,36; Smp. 80- 83°);
N-n-Hexadecanoyl-Saframycin $Y_3$ (Rf: 0,43, 0,36; Smp. 76- 80°);
N-n-Octadecanoyl-Saframycin $Y_3$ (Rf: 0,44, 0,36; Smp. 60- 65°);
N-Benzoyl-Saframycin $Y_3$ (Rf: 0,36, 0,28; Smp. 127-130°);
N-p-Brombenzoyl-Saframycin $Y_3$ (Rf: 0,38, 0,27; Smp. 151-156°);
N-Cinnamoyl-Saframycin $Y_3$ (Rf: 0,36, 0,27; Smp. 146-150°);
N-Oxalyl-Saframycin $Y_3$ (Rf: 0,31, 0,20; Smp. 200-207° Zers.);
N-p-Toluolsulfonyl-Saframycin $Y_3$ (Rf: 0,43, 0,33; Smp. 137-140°);
hergestellt durch Reaktion von 280 mg (0,5 mMol) Saframycin $Y_3$ mit 2,0 mMol des entsprechenden Säurechlorids in Gegenwart von 253 mg (2,5 mMol) Triäthylamin. Rf-Werte werden für Benzol/Essigsäureäthylester 1:2 und 1:1 angegeben.

Beispiel 8: Analog Beispiel 6 werden
N-n-Hexanoyl-Saframycin $Y_{d-I}$ (Rf: 0,45; Smp. 116-119°) und
N-Pivaloyl-Saframycin $Y_{d-I}$ (Rf: 0,43; Smp. 158-162° Zers.)
durch Reaktion von 280 mg (0,5 mMol) Saframycin $Y_{d-I}$ mit 325 mg (2,0 mMol) n-Hexanoylchlorid bzw. 250 mg (2,0 mMol) Pivaloylchlorid in Gegenwart von 253 mg (2,5 mMol) Triäthylamin hergestellt. Rf-Werte werden für Benzol/Essigsäureäthylester 1:2 angegeben.

Beispiel 9: N-Phenylcarbamoyl-Saframycin $Y_3$

280 mg (0,5 mMol) Saframycin $Y_3$ werden in 40 ml wasserfreiem Benzol gelöst und mit 595 mg (5,0 mMol) Phenylisocyanat versetzt. Die Reaktionsmischung wird bei 40-50° eine Stunde lang gerührt, dann in wässrige 0,05 N Natriumcarbonat-Lösung gegossen und mit 200 ml Essigsäureäthylester extrahiert. Die organische Phase wird mit Wasser gewaschen und über wasserfreiem Natriumsulfat getrocknet. Das Lösungsmittel wird abgedampft und der Rückstand mit 50 ml Benzol extrahiert. Das Benzol wird abgedampft und der Rückstand durch Säulenchromatographie Kieselgel 60 (Merck Deutschland) mit Benzol und Essigsäureäthylester 4:1, 2:1, 1:1 als Laufmittel gereinigt. Das Produkt wird weiter durch präparative Kieselgel-Dünnschichtchromatographie gereinigt. Rf: 0,39 (Benzol/Essigsäureäthylester 1:4), 0,49 (Chloroform/Aethanol 20:1); Smp. 162-166°.

Beispiel 10: Analog Beispiel 9 werden
N-Phenylthiocarbamoyl-Saframycin $Y_3$ (Rf: 0,49, 0,59; Smp. 155-159°);
N-α-Naphthylcarbamoyl-Saframycin $Y_3$ (Rf: 0,43, 0,51; Smp. 161-164°);
durch Reaktion von 280 mg (0,5 mMol) Saframycin $Y_3$ mit 676 mg (5,0 mMol) Phenylisothiocyanat bzw. 846 mg (5,0 mMol) α-Naphthylisocyanat hergestellt. Rf-Werte werden für Essigsäureäthylester/Benzol 4:1 und Chloroform/Aethanol 20:1 angegeben.

Beispiel 11: Lacktabletten enthaltend 10 mg N-Pivaloyl-Saframcyin $Y_3$ werden folgendermassen hergestellt:

Zusammensetzung für 10 000 Tabletten:
N-Pivaloyl-Saframycin $Y_3$ 100,0 g
Maisstärke 680,0 g
kolloidale Kieselsäure 200,0 g
Magnesiumstearat 20,0 g
Stearinsäure 50,0 g
Natriumcarboxymethyl-Stärke 250,0 g
Wasser q.s.

Eine Mischung von N-Pivaloyl-Saframycin $Y_3$, 50 g Maisstärke und kolloidale Kieselsäure werden mit Stärkepaste bestehend aus 250 g Maisstärke und 2,2 kg entmineralisiertem Wasser zu einer feuchten

Mischung verarbeitet. Diese Zusammensetzung wird durch ein Sieb mit 3 mm Maschenweite getrieben und 30 Minuten bei 45° in einem Wirbelschichttrockner getrocknet. Das trockene Granulat wird durch ein Sieb mit 1 mm Maschenweite getrieben, mit einer vorher gesiebten Mischung (1 mm-Sieb) von 330 g Maisstärke, Magnesiumstearat, Stearinsäure und Natriumcarboxymethyl-Stärke gemischt und zu schwach gewölbten Tabletten gepresst. Die Tabletten werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid gleichmässig während 30 Minuten besprüht. Die Tabletten werden gleichzeitig durch Einblasen von warmer Luft von 60° getrocknet.

Es ist auch möglich, anstelle der in diesem Beispiel genannten Verbindung die gleiche Menge irgendeiner Verbindung der anderen vorstehenden Beispiele zu verwenden.

Beispiel 12: Test für in vitro-Zytotoxizität

Die Wirksamkeit gegen Tumoren in vitro wird mit einer kultivierten Leukämie-Zellinie L1210 (K. Kishi et al., J. Antibiot. 37, 847 (1984)) bestimmt. Die Zellen werden in Eagle's minimum essential medium (Nissui Seiyaku Co. Ltd., Tokyo) enthaltend 10 % fötales Kälberserum (Gibco Laboratories) in Suspension-Kultur gehalten. Die Zellen werden bei einer Konzentration von $3x10^4$ Zellen/ml in 24-Loch-Mikrotiterplatten (Costar) suspendiert und bei 37° in einer feuchten Atmosphäre enthaltend 5 % $CO_2$ in Luft 24 Stunden lang kultiviert, um exponentielles Wachstum auszulösen. Diese Kulturen werden mit einer Reihe von jeweils um den Faktor 10 verdünnten Proben Saframycin-Derivaten dieser Erfindung versetzt. Die Konzentration der Zellen wird nach 3-tägiger Inkubation durch elektrische Zählung mit einem Sysmex CC-130 Mikro-Zellzählgerät (Toa Electronics Ltd., Tokyo) bestimmt. Die Wachstumshemmung (Inhibierung I) in Prozent wird für jede Dosismenge nach der Formel I = (C3-T3)/(C3-C0)x100, worin T3 die Zellkonzentration in den Vertiefungen der Mikrotiterplatte bei einer gegebenen Verdünnung des Wirkstoffs nach 3-tägiger Inkubation, C3 die Zellkonzentration in der entsprechenden Kontroll-Inkubation und C0 die Zellkonzentration in den Vertiefungen vor der 3-tägigen Inkubation bedeuten, berechnet. $ID_{50}$ (50 % Hemmwirkdosis) wird durch Interpolierung bestimmt.

Die folgenden $ID_{50}$-Werte (µg/ml) werden gefunden:

N,N-Diäthyl-Saframycin $Y_3$ 0,0035
N-Propyl-Saframycin $Y_3$ 0,002
N,N-Dipropyl-Saframycin $Y_3$ 0,0015
N-Isopropyl-Saframycin $Y_3$ 0,0018
N-Butyl-Saframycin $Y_3$ 0,009
N,N-Dibutyl-Saframycin $Y_3$ 0,08
N-p-Nitrobenzyl-Saframycin $Y_3$ 0,0015
N-Vanillyl-Saframycin $Y_3$ 0,005
N-Acetyl-Saframycin $Y_3$ 0,008
N-Trifluoracetyl-Saframycin $Y_3$ 0,006
N-Propionyl-Saframycin $Y_3$ 0,02
N-Butyryl-Saframycin $Y_3$ 0,014
N-Pivaloyl-Saframycin $Y_3$ 0,01
N-Valeryl-Saframycin $Y_3$ 0,023
N-n-Hexanoyl-Saframycin $Y_3$ 0,0075
N-n-Octanoyl-Saframycin $Y_3$ 0,11
N-n-Decanoyl-Saframycin $Y_3$ 0,11
N-n-Dodecanoyl-Saframycin $Y_3$ 0,023
N-n-Tetradecanoyl-Saframycin $Y_3$ 0,025
N-n-Hexadecanoyl-Saframycin $Y_3$ 0,03
N-n-Octadecanoyl-Saframycin $Y_3$ 0,075
N-Benzoyl-Saframycin $Y_3$ 0,009
N-p-Brombenzyol-Saframycin $Y_3$ 0,01
N-Phenylthiocarbamoyl-Saframycin $Y_3$ 0,1
N-Phenylcarbamoyl-Saframycin $Y_3$ 0,04
N-α-Naphthylcarbamoyl-Saframycin $Y_3$ 0,08

Beispiel 13: In vivo-Zytotoxizität

$10^5$ L1210 Leukämie-Zellen werden intraperitoneal in 6 Wochen alte männliche $BDF_1$-Mäuse (Shizuoka Laboratory Animal Center) implantiert. Die Saframycin-Derivate der vorliegenden Erfindung werden bei Konzentrationen von 1,0 bis 5,0 mg/kg suspendiert in 0,2 ml Kochsalzlösung/0,25 % Carboxymethylcellulose 24 Stunden nach Implantation des Tumors einmal täglich von Tag 1 bis Tag 9 injiziert. Die Verlängerung der Lebensdauer wird durch Vergleich der mittleren Ueberlebenszeit (Tage) von behandelten Mäusen (T) mit derjenigen von nichtbehandelten Mäusen (C) bestimmt, d.h. Prozentzunahme der Lebensdauer (increase in life span) (ILS) = (T/C-1)x100.

Die folgenden Werte werden erhalten:

| | Dosis (mg/kg) | mittlere Ueber-lebenszeit (Tage, T/C) | ILS (%) |
|---|---|---|---|
| N-Acetyl-Saframycin $Y_3$ | 3,0 | 12,5/10,2 | 23 |
| N-Trifluoracetyl-Saframycin $Y_3$ | 3,0 | 10,5/10,2 | 3 |
| N-Propionyl-Saframycin $Y_3$ | 4,0 | 12,5/10,2 | 23 |
| N-Butyryl-Saframycin $Y_3$ | 3,0 | 12,5/10,2 | 23 |
| N-Pivaloyl-Saframycin $Y_3$ | 3,0 | 13,2/10,2 | 29 |
| N-Valeryl-Saframyin $Y_3$ | 5,0 | 10,5/10,5 | 0 |
| N-n-Hexanoyl-Saframycin $Y_3$ | 3,0 | 13,5/10,2 | 32 |
| N-n-Octanoyl-Saframycin $Y_3$ | 5,0 | 12,5/11,1 | 13 |
| N-n-Decanoyl-Saframycin $Y_3$ | 5,0 | 12,0/11,1 | 10 |
| N-n-Dodecanoxyl-Saframycin $Y_3$ | 5,0 | 11,5/11,1 | 4 |
| N-n-Tetradecanoxyl-Saframycin $Y_3$ | 5,0 | 12,2/11,1 | 10 |
| N-n-Hexadecanoyl-Saframycin $Y_3$ | 4,0 | 11,5/10,2 | 13 |
| N-n-Octadecanoyl-Saframycin $Y_3$ | 4,0 | 12,0/10,2 | 18 |
| N-Benzyol-Saframycin $Y_3$ | 3,0 | 11,5/10,2 | 13 |
| N-p-Bromobenzyol-Saframycin $Y_3$ | 3,0 | 12,5/10,2 | 23 |

**Patentansprüche**

1. Verbindung der Formel

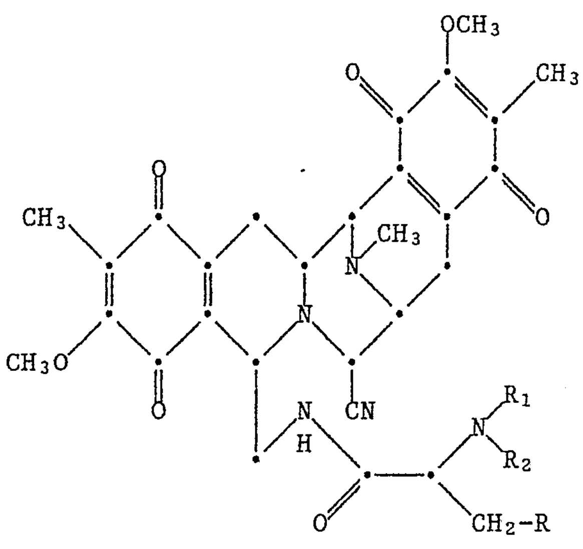

(II),

worin R Wasserstoff oder $C_1$-$C_4$-Alkyl, $R_1$ $C_1$-$C_7$-Alkyl, Aryl-$C_1$-$C_4$-alkyl oder die Acylgruppe einer organischen Säure und $R_2$ Wasserstoff oder $C_1$-$C_7$-Alkyl darstellen, und Salze dieser Verbindungen.

2. Verbindung der Formel II gemäss Anspruch 1, worin R Wasserstoff oder $C_1$-$C_4$-Alkyl, $R_1$ $C_1$-$C_7$-Alkyl oder Aryl-$C_1$-$C_4$-alkyl und $R_2$ Wasserstoff oder $C_1$-$C_7$-Alkyl darstellen, und Salze dieser Verbindungen.

3. Verbindung der Formel II gemäss Anspruch 1, worin R Wasserstoff oder $C_1$-$C_4$-Alkyl, $R_1$ die Acylgruppe einer organischen Säure und $R_2$ Wasserstoff bedeuten, und Salze dieser Verbindungen.

4. Verbindung der Formel II gemäss Anspruch 1 oder 2, worin R Wasserstoff oder Methyl, $R_1$ $C_1$-$C_4$-Alkyl, Benzyl oder durch Nitro, Amino, Halogen, Hydroxy und/oder $C_1$-$C_4$-Alkoxy substituiertes Benzyl und $R_2$ Wasserstoff oder $C_1$-$C_4$-Alkyl darstellen, und pharmazeutisch verwendbare Säureadditionssalze dieser Verbindungen.

5. Verbindung der Formel II gemäss Anspruch 1 oder 3, worin die Acylgruppe $R_1$ eine Acylgruppe einer Carbonsäure, eines Kohlensäurehalbesters, einer N-substituierten oder N,N-disubstituierten Carbaminsäure oder Thiocarbaminsäure oder einer Sulfonsäure darstellt, und pharmazeutisch verwendbare Säureadditionssalze dieser Verbindungen.

6. Verbindung der Formel II gemäss Anspruch 1 oder 3, worin R Wasserstoff oder Methyl, $R_1$ $C_1$-$C_7$-Alkanoyl, geradkettiges $C_8$-$C_{20}$-Alkanoyl mit einer geraden Anzahl Kohlenstoffatome, durch Halogen substituiertes $C_2$-$C_7$-Alkanoyl, Benzoyl, durch Nitro, Amino, Halogen und/oder $C_1$-$C_4$-Alkoxy substituiertes Benzoyl, Phenylcarbamoyl, 1-oder 2-Naphthylcarbamoyl oder Phenylthiocarbamoyl und $R_2$ Wasserstoff bedeuten, und pharmazeutisch verwendbare Säureadditionssalze dieser Verbindungen.

7. Verbindung der Formel II gemäss Anspruch 1 oder 2, worin R Wasserstoff, $R_1$ $C_1$-$C_4$-Alkyl, Benzyl oder durch Nitro, Hydroxy und/oder $C_1$-$C_4$-Alkoxy substituiertes Benzyl und $R_2$ Wasserstoff oder $C_1$-$C_4$-Alkyl bedeuten, und pharmazeutisch verwendbare Säureadditionssalze dieser Verbindungen.

8. Verbindung der Formel II gemäss Anspruch 1 oder 3, worin R Wasserstoff oder Methyl, $R_1$ $C_1$-$C_7$-Alkanoyl, geradkettiges $C_8$-$C_{18}$-Alkanoyl mit einer geraden Anzahl Kohlenstoffatome, durch Halogen substituiertes $C_2$-$C_7$-Alkanoyl, Benzoyl oder durch Nitro, Amino, Halogen und/oder $C_1$-$C_4$-Alkoxy substituiertes Benzoyl und $R_2$ Wasserstoff bedeuten, und pharmazeutisch verwendbare Säureadditionssalze dieser Verbindungen.

9. Verbindung der Formel I gemäss Anspruch 1 oder 3, worin R Wasserstoff oder Methyl, $R_1$ Acetyl, Propionyl, n-Butyryl, Isobutyryl, n-Valeryl, Pivaloyl, n-Hexanoyl, n-Octanoyl, n-Decanoyl, n-Dodecanoyl, n-Tetradecanoyl, n-Hexadecanoyl, n-Octadecanoyl, Trifluoracetyl, Benzoyl oder p-Brombenzoyl und $R_2$ Wassserstoff bedeuten, und pharmazeutisch verwendbare Säureadditionssalze dieser Verbindungen.

10. N,N-Diäthyl-Saframycin $Y_3$ gemäss Anspruch 7.

11. N,N-Di-n-propyl-Saframycin $Y_3$ gemäss Anspruch 7.

12. N-Pivaloyl-Saframycin $Y_3$ gemäss Anspruch 9.

13. N-n-Hexanoyl-Saframycin $Y_3$ gemäss Anspruch 9.

14. N-Pivaloyl-Saframycin $Y_{d-I}$ gemäss Anspruch 9.

15. N-n-Hexanoyl-Saframycin $Y_{d-I}$ gemäss Anspruch 9.

16. Verfahren zur Herstellung einer Verbindung der Formel II gemäss Anspruch 1, dadurch charakterisiert, dass

a) eine Verbindung der Formel

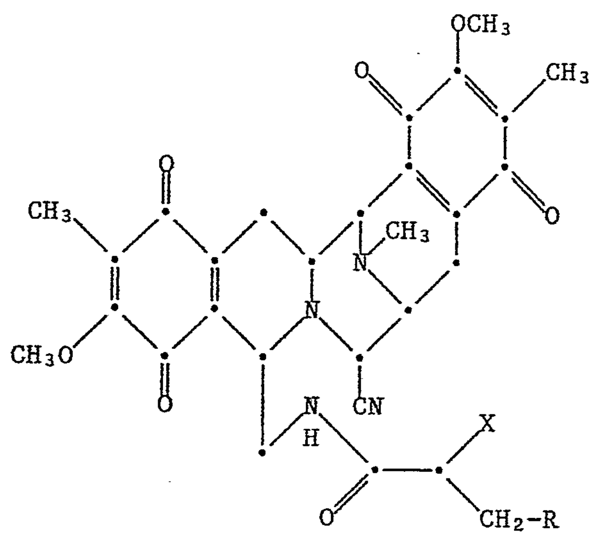

(III),

worin R Wasserstoff oder $C_1$-$C_4$-Alkyl und X eine in eine Gruppe $-NR_1R_2$ überführbare Gruppe darstellen, mit einem Reagens behandelt wird, das die Gruppe $-NR_1R_2$ einführen kann, oder

b) eine Verbindung der Formel

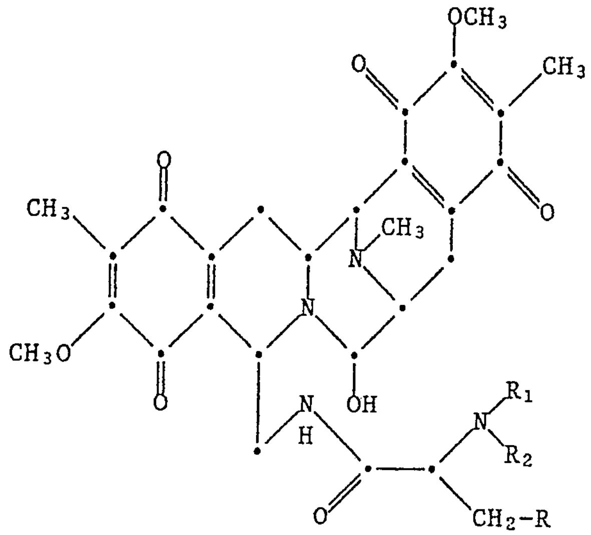

(IV),

worin R, $R_1$ und $R_2$ wie unter Formel II definiert sind, mit Cyanwasserstoff oder einem Salz von Cyanwasserstoff umgesetzt wird,
und, wenn erwünscht, eine erhältliche Verbindung der Formel II in ihr Säureadditionssalz oder ein erhältliches Salz in die freie Verbindung oder in ein anderes Säureadditionssalz umgewandelt wird und/oder eine Mischung von Isomeren in die einzelnen Isomeren aufgetrennt wird.

17. Pharmazeutische Zusammensetzung enthaltend eine wirksame Menge einer Verbindung der Formel II gemäss Anspruch 1 oder ein pharmazeutisch verwendbares Säureadditionssalz davon im Gemisch mit einem pharmazeutisch verwendbaren Träger.

Patentansprüche für die folgenden Vertragsstaaten: AT, ES, GR

1. Verfahren zur Herstellung von Verbindungen der Formel

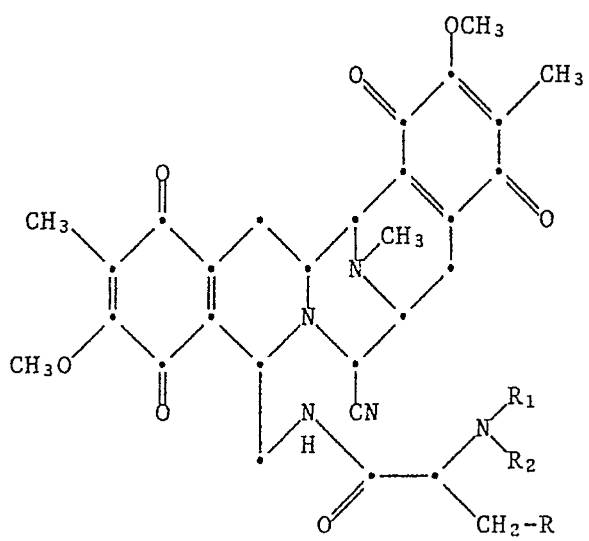

(II),

worin R Wasserstoff oder $C_1$-$C_4$-Alkyl, $R_1$ $C_1$-$C_7$-Alkyl, Aryl-$C_1$-$C_4$-alkyl oder die Acylgruppe einer organischen Säure und $R_2$ Wasserstoff oder $C_1$-$C_7$-Alkyl darstellen, und von Salzen dieser Verbindungen, dadurch charakterisiert, dass
a) eine Verbindung der Formel

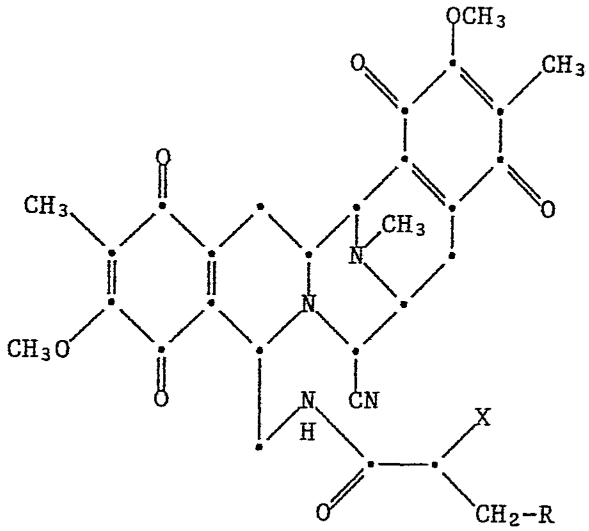

(III),

worin R Wasserstoff oder $C_1$-$C_4$-Alkyl und X eine in eine Gruppe -$NR_1R_2$ überführbare Gruppe darstellen, mit einem Reagens behandelt wird, das die Gruppe -$NR_1R_2$ einführen kann, oder
b) eine Verbindung der Formel

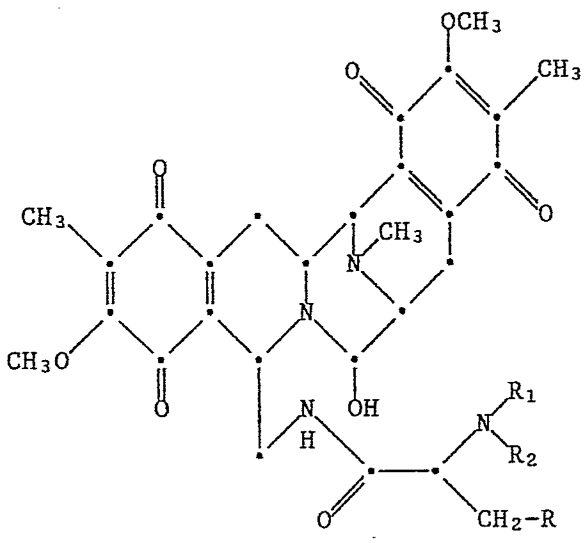

(IV),

worin R, $R_1$ und $R_2$ wie unter Formel II definiert sind, mit Cyanwasserstoff oder einem Salz von Cyanwasserstoff umgesetzt wird,
und, wenn erwünscht, eine erhältliche Verbindung der Formel II in ihr Säureadditionssalz oder ein erhältliches Salz in die freie Verbindung oder in ein anderes Säureadditionssalz umgewandelt wird und/oder eine Mischung von Isomeren in die einzelnen Isomeren aufgetrennt wird.

2. Verfahren gemäss Anspruch 1 zur Herstellung von Verbindungen der Formel II, worin R Wasserstoff oder $C_1$-$C_4$-Alkyl, $R_1$ $C_1$-$C_7$-Alkyl oder Aryl-$C_1$-$C_4$-alkyl und $R_2$ Wasserstoff oder $C_1$-$C_7$-Alkyl darstellen, und von Salzen dieser Verbindungen.

3. Verfahren gemäss Anspruch 1 zur Herstellung von Verbindungen der Formel II, worin R Wasserstoff oder $C_1$-$C_4$-Alkyl, $R_1$ die Acylgruppe einer organischen Säure und $R_2$ Wasserstoff bedeuten, und von Salzen dieser Verbindungen.

4. Verfahren gemäss Anspruch 1 oder 2 zur Herstellung von Verbindungen der Formel II, worin R Wasserstoff oder Methyl, $R_1$ $C_1$-$C_4$-Alkyl, Benzyl oder durch Nitro, Amino, Halogen, Hydroxy, und/oder $C_1$-$C_4$-Alkoxy substituiertes Benzyl und $R_2$ Wasserstoff oder $C_1$-$C_4$-Alkyl darstellen, und von pharmazeutisch verwendbaren Säureadditionssalzen dieser Verbindungen.

5. Verfahren gemäss Anspruch 1 oder 3 zur Herstellung von Verbindungen der Formel II, worin die Acylgruppe $R_1$ eine Acylgruppe einer Carbonsäure, eines Kohlensäurehalbesters, einer N-substituierten oder N,N-disubstituierten Carbaminsäure oder Thiocarbaminsäure oder einer Sulfonsäure darstellt, und

von pharmazeutisch verwendbaren Säureadditionssalzen dieser Verbindungen.

6. Verfahren gemäss Anspruch 1 oder 3 zur Herstellung von Verbindungen der Formel II, worin R Wasserstoff oder Methyl, $R_1$ $C_1$-$C_7$-Alkanoyl, geradkettiges $C_8$-$C_{20}$-Alkanoyl mit einer geraden Anzahl Kohlenstoffatome, durch Halogen substituiertes $C_2$-$C_7$-Alkanoyl, Benzoyl, durch Nitro, Amino, Halogen und/oder $C_1$-$C_4$-Alkoxy substituiertes Benzoyl, Phenylcarbamoyl, 1-oder 2-Naphthylcarbamoyl oder Phenylthiocarbamoyl und $R_2$ Wasserstoff bedeuten, und von pharmazeutisch verwendbaren Säureadditionssalzen dieser Verbindungen.

7. Verfahren gemäss Anspruch 1 oder 2 zur Herstellung von Verbindungen der Formel II, worin R Wasserstoff, $R_1$ $C_1$-$C_4$-Alkyl, Benzyl oder durch Nitro, Hydroxy und/oder $C_1$-$C_4$-Alkoxy substituiertes Benzyl und $R_2$ Wasserstoff oder $C_1$-$C_4$-Alkyl bedeuten, und von pharmazeutisch verwendbaren Säureadditionssalzen dieser Verbindungen.

8. Verfahren gemäss Anspruch 1 oder 3 zur Herstellung von Verbindungen der Formel II, worin R Wasserstoff oder Methyl, $R_1$ $C_1$-$C_7$-Alkanoyl, geradkettiges $C_8$-$C_{18}$-Alkanoyl mit einer geraden Anzahl Kohlenstoffatome, durch Halogen substituiertes $C_2$-$C_7$-Alkanoyl, Benzoyl oder durch Nitro, Amino, Halogen und/oder $C_1$-$C_4$-Alkoxy substituiertes Benzoyl und $R_2$ Wasserstoff bedeuten, und von pharmazeutisch verwendbaren Säureadditionssalzen dieser Verbindungen.

9. Verfahren gemäss Anspruch 1 oder 3 zur Herstellung von Verbindungen der Formel II, worin R Wasserstoff oder Methyl, $R_1$ Acetyl, Propionyl, n-Butyryl, Isobutyryl, n-Valeryl, Pivaloyl, n-Hexanoyl, n-Octanoyl, n-Decanoyl, n-Dodecanoyl, n-Tetradecanoyl, n-Hexadecanoyl, n-Octadecanoyl, Trifluoracetyl, Benzoyl oder p-Brombenzoyl und $R_2$ Wassserstoff bedeuten, und von pharmazeutisch verwendbaren Säureadditionssalzen dieser Verbindungen.

10. Verfahren gemäss Anspruch 7 zur Herstellung von N,N-Diäthyl-Saframycin $Y_3$.

11. Verfahren gemäss Anspruch 7 zur Herstellung von N,N-Di-n-propyl-Saframycin $Y_3$.

12. Verfahren gemäss Anspruch 9 zur Herstellung von N-Pivaloyl-Saframycin $Y_3$.

13. Verfahren gemäss Anspruch 9 zur Herstellung von N-n-Hexanoyl-Saframycin $Y_3$.

14. Verfahren gemäss Anspruch 9 zur Herstellung von N-Pivaloyl-Saframycin $Y_{d-I}$.

15. Verfahren gemäss Anspruch 9 zur Herstellung von N-n-Hexanoyl-Saframycin $Y_{d-I}$.

16. Verfahren zur Herstellung pharmazeutischer Zusammensetzungen enthaltend eine wirksame Menge einer Verbindung der Formel II gemäss Anspruch 1 oder ein pharmazeutisch verwendbares Säureadditionssalz davon im Gemisch mit einem pharmazeutisch verwendbaren Träger, dadurch gekennzeichnet, dass man die Verbindung der Formel II oder ein Salz davon mit einem Trägermaterial mischt.

Europäisches Patentamt

# EUROPÄISCHER RECHERCHENBERICHT

Nummer der Anmeldung

EP 87 81 0083

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | WO-A-8 501 049 (YOSHITOMI)<br>* Zusammenfassung *<br>--- | 1,17 | C 07 D 471/18<br>A 61 K 31/495//<br>(C 07 D 471/18<br>C 07 D 241:00<br>C 07 D 221:00<br>C 07 D 221:00 ) |
| Y | CHEMICAL ABSTRACTS, Band 103, 1985, Seite 378, Zusammenfassung Nr. 119437h, Columbus, Ohio, US; T. ARAI et al.: "Directed biosynthesis of new saframycin derivatives with resting cells of Streptomyces lavendulae", & ANTIMICROB. AGENTS CHEMOTHER. 1985, 28(1), 5-11<br>* Zusammenfassung und Chemical Substance Index, Seite 1460, Spalte 2, Zeilen 63-70 und Seite 5765, Spalte 1, Zeilen 1-8 *<br>--- | 1,17 | |
| P,X | CHEMICAL ABSTRACTS, Band 106, 1987, Seite 15, Zusammenfassung Nr. 27369w, Columbus, Ohio, US; S. KANEDO et al.: "Antitumor activity of new semisynthetic saframycin derivatives", & JPN. J. CANCER RES. (GANN) 1986, 77(10), 1043-9<br>* Zusammenfassung *<br>----- | 1,17 | RECHERCHIERTE SACHGEBIETE (Int. Cl.4)<br>C 07 D 71/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 21-05-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82